# EUROPEAN PATENT APPLICATION

(11) **EP 4 302 775 A1**
(43) Date of publication of application: **10.01.2024**
(21) Application number: 22762350.1
(22) Date of filing: 07.02.2022
(51) Int. Cl.: A61K 38/17, A61P 25/22, A61P 25/24

(54) **APPLICATION OF INSULIN-LIKE GROWTH FACTOR-BINDING PROTEIN 2 IN PREPARATION OF DRUGS FOR RESISTING MENTAL DISORDERS CAUSED BY EARLY LIFE STRESS**

(30) Priority: 04.03.2021 CN 202110241069; 24.03.2021 CN 202110316213
(71) Applicant: Shanghai Mental Health Center (Shanghai Psychological Counselling Training Center), Shanghai 200030 (CN)
(72) Inventor: WANG, Zhen, Shanghai 200030 (CN); SHI, Dongdong, Shanghai 200030 (CN); ZHANG, Yingdan, Shanghai 200030 (CN)
(74) Representative: Dummett Copp LLP
(86) International application number: PCT/CN2022/075358
(87) International publication number: WO 2022/183883

(57) **Abstract**

An application of insulin-like growth factor-binding protein 2 (IGFBP2) in the preparation of drugs for resisting mental disorders caused by early-life stress. IGFBP2 puberty intervention and adult intervention can significantly improve anxiety and depression behaviors caused by early life stress.

## Description

### Cross referencing of related applications

This application claims the priority of the Chinese invention patent application with application number 202110241069.3 filed on March 4, 2021 and application number 202110316213.5 filed on March 24, 2021, and its content is incorporated into this application by reference.

### Field of the invention

The present invention relates to the field of pharmaceutical technology, in particular to the application of insulin-like growth factor-binding protein 2 in the preparation of drugs for resisting mental disorders caused by early-life stress.

### Background of the invention

In 2019, the "China Health Survey" conducted by the China's National Health Commission and the Ministry of Science and Technology showed that mental diseases accounted for about 20% of the total burden of diseases in China, ranking first, with 16.57% (about 230 million) of individuals who had suffered from mental disorders. Many mental illnesses stem from experiences of stress. In childhood, due to the immaturity of physical and psychological factors, one is more susceptible to stress damage, resulting in abnormal mental behavior. Childhood stress events, including parents' absence, parents' neglect, physical or mental abuse, can significantly enhance the sensitivity of adults to stress and increase the incidence rate of mental diseases in adults. Early-life stress is significantly associated with depression, anxiety, and post-traumatic stress disorder. And epidemiological data shows that the impact of early-life stress on cognitive and emotional function is lifelong. Clinical and animal experimental studies have shown that early-life psychic trauma increases the susceptibility to poststress depression in adults, leading to increased levels of anxiety and depression.

The US national survey found that about 2.1% -8% of adolescents were diagnosed with depression. Depression often coexists with adult obesity, diabetes, cardiovascular diseases, and autoimmune diseases, resulting in poor treatment compliance and drug responsiveness, high medical costs, and serious impact on families and society. Drug therapy for depression can only be effective in 60% of patients in the early-life stage, so there is an urgent need to research and explore new intervention drugs to improve the cure rate.

Research suggests that the activation of growth factor receptors is beneficial for the treatment of post traumatic stress disorder (PTSD). The FDA has approved insulin like growth factor 1 (IGF1) as a drug for treating congenital dwarfism, and studies have shown that IGF1 has effects of anti-anxiety and depression. Insulin growth factor-binding protein 2 (IGFBP2) is one of the six major members of IGF binding proteins. Most of IGFBP2 is synthesized and released in the liver. IGFBP2 is widely present in the central system and highly expressed in the developing brain, secreted in primary astrocytes and neurons. Its concentration in the periphery is relatively low, and the molecular weight of IGFBP2 is 36 kD, which can cross the blood brain barrier (BBB). At present, there is no relevant research proving that IGFBP2 can treat anxiety and depression-like behavior caused by early-life stress.

### Summary of the invention

The purpose of the present invention is to overcome the shortcomings of the prior art and provide an application of insulin-like growth factor-binding protein 2 in the preparation of drugs for resisting mental disorders caused by early-life stress.

The technical solution provided by the present invention specifically relates to the application of insulin-like growth factor-binding protein 2 in the preparation of drugs for resisting mental disorders caused by early-life stress.

Preferably, the mental disorders comprises anxiety and depression-like behavior.

Specifically, IGFBP2 is mainly used in the treatment of mental disorders caused by early-life stress. Anti-mental disorders mainly comprises anti-depressant and anti-anxiety. The mental disorders caused by early-life stress comprises: loss of pleasure caused by early-life stress; anxiety-like behavior caused by early-life stress.

The intervention time of IGFBP2 comprises Puberty intervention and Adult intervention. IGFBP2 intervention in adolescence and adulthood can significantly improve depression and anxiety-like behavior caused by early-life stress.

### Brief Description Of The Drawings

Figure 1 is a flowchart for verifying the role of IGFBP2 in early-life stress-induced psychic trauma.
Figures 2a to 2c show the effect of IGFBP2 intervention in adolescence on anxiety and depression-like behavior in animals of adulthood.
Figures 3a to 3b show the effect of IGFBP2 intervention in adolescence on the levels of adrenocorticotropic hormone in animals of adulthood undergoing stress.
Figures 4a to 4c show the effect of IGFBP2 intervention in adulthood on anxiety and depression-like behavior in animals of adulthood.

### Detailed description of the invention

In order to better describe the technical content of the present invention and verify the role of IGFBP2 in early-life stress-induced psychic trauma, further description will be given in conjunction with specific embodiments below.

### Example 1

### Establishing an early-life stress model

SD rats are raised in a 12 hour light/dark rhythm environment and can freely access water and food. All ethology assessment were conducted in the light rhythm of animals. Farrowing: SD pregnant mice are raised in a single cage 1-3 days before farrowing. On the day of farrowing (PND 0), the number of farrowing, gender, and weight are recorded, and the cage is changed on the day of farrowing. During lactation, the cage is not changed. The lactation period is 21 days, and PND 21 separates the offspring and mother mice. The offspring are divided into male and female cages, with 3-5 mice per cage.

The early-life stress method chosen was maternal-infant separation, with PND 4-21 offspring and mother mice separated for 4 hours per day. During the separation period, each offspring was separated from each other and the offspring and mother mice were in different rooms. During the separation period, ensure that the environmental temperature of the offspring is between 27 and 33 °C, and the survival rate of the offspring is not affected by the separation. After separation, the motor ability, feeding and water intake ability of the offspring are not affected. During the experiment, the weight of offspring mice was measured at PND 0, PND 7, PND 14, PND 21, PND 28, PND 45, and PND 56, respectively.

The established early-life stress model is used for IGFBP2 intervention.

### Example 2

### Puberty intervention

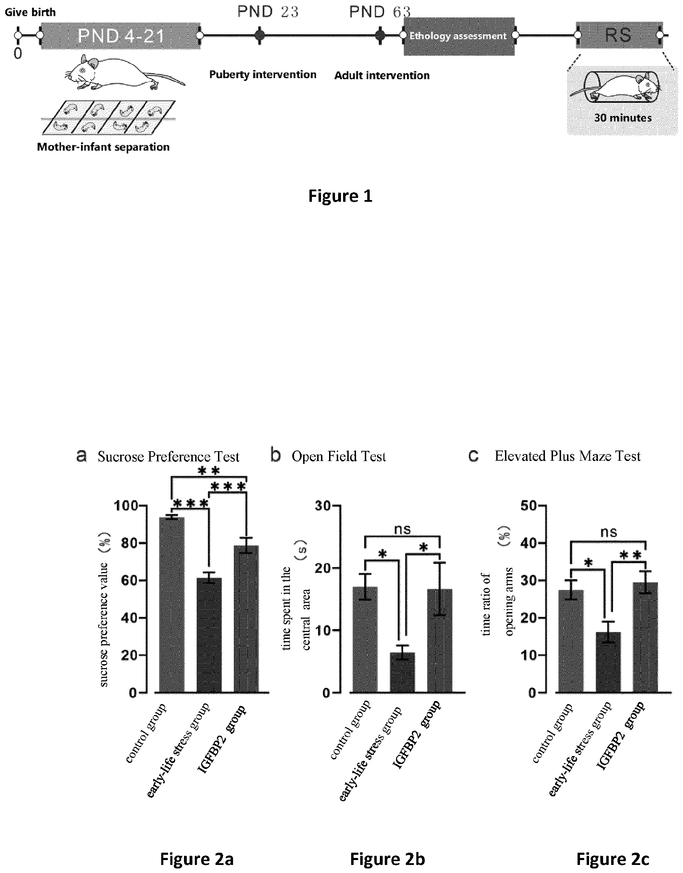

The animals were divided into three groups: the control group (normal feeding group), the mother-infant separation group, and the IGFBP2 intervention in adolescence group. The number of animals in three groups is greater than or equal to 10. As shown in Figure 1, the intervention time of IGFBP2 in adolescence is PND 28 (note, PND is the number of days after birth), and the intervention method is tail vein injection. The intervention dose is 1 µ g/kg, and the intervention frequency is 1 time. After the intervention, the animals were placed back in the breeding cage and subjected to ethology assessment at 9 weeks of age. RS in Figure 1 shows restraint stress.

Ethology assessment is used to verify the intervention effect of IGFBP2 on psychic trauma caused by early-life stress. All ethology assessments were conducted during rat light exposure. Before conducting ethology assessments, the rats were placed in a behavioral room and acclimated for 2 hours. During each experiment, wipe each mouse with 70% alcohol to avoid the impact of odor on the next mouse. Handle rats for a few minutes every day before ethology assessment, and adapt for at least 4 days. Ethology assessment include Sucrose Preference Test for evaluating depression-like behavior, as well as, Open Field Test and Elevated Plus Maze Test for evaluating anxiety-like behavior.

To further determine the intervention effect of IGFBP2 on anxiety and depression-like behavior caused by early-life stress, changes in serum corticosterone levels were detected.

Firstly, Sucrose Preference Test was conducted to detect depression-like behavior in animals, followed by Open Field Test and Elevated Plus Maze Test to detect anxiety-like behavior in animals. As shown in Figures 2a to 2c, the impact of IGFBP2 intervention in adolescence on anxiety and depression-like behavior in animals of adulthood is shown. Figure 2a shows Sucrose Preference Test, with sucrose preference value%=sucrose consumption/ ( sucrose consumption + water consumption) x100; Figure 2b shows Open Field Test, with a total time of 10 minutes and the analysis data shows the time the animals spent in the central area; Figure 2c shows Elevated Plus Maze Test, with an open arm time ratio %=open arm time/(open arm time + closed arm time) x100. One-way ANOVA, *** represents P<0.001, ** represents P<0.001, and * represents P<0.05.

As shown in Figure 2a, the results of Sucrose Preference Test showed that the sucrose preference values (61.37%) of animals in the mother-infant separation group (early-life stress group) were significantly lower than those in the normal feeding group (93.88%, One-way ANOVA, P<0.001), while the sucrose preference values (78.73%) of animals in the IGFBP2 intervention group were significantly higher than those in the mother-infant separation group (P<0.001), but still significantly lower than the animals in the normal feeding group (93.88%, One-way ANOVA, P=0.003). This result indicates that IGFBP2 can significantly improve depression-like behavior caused by early-life stress.

As shown in Figure 2b, the results of Open Field Test showed that the time (6.416 s)spent in the central area of the mother-infant separation group (early-life stress group) was significantly lower than that of the normal feeding group (16.970 s, One-way ANOVA, P=0.032). After the IGFBP2 intervention, the time spent in the central area of the mother-infant separation group was significantly increased (16.65 s, One-way ANOVA, P=0.039), and there was no difference between the mother-infant separation group and the normal feeding group (P=0.996). This result indicates that IGFBP2 intervention in adolescence can significantly improve anxiety-like behavior caused by mother-infant separation.

In addition, comparing the motor distance of the three groups of animals, it was found that there was no significant difference in motor distance between the three groups, indicating that early-life stress and IGFBP2 intervention did not affect the motor function of the animals.

As shown in Figure 2c, the results of Elevated Plus Maze Test showed that the time ratio of opening arms in the normal feeding group (27.47%) was significantly longer than that in the mother-infant separation group (early-life stress group, 16.18%), and the IGFBP2 intervention significantly improved the time of opening arms in mother-infant separation animals (29.49%, One-way ANOVA, P=0.005). This result further confirms that IGFBP2 can effectively improve anxiety-like behavior caused by early-life stress.

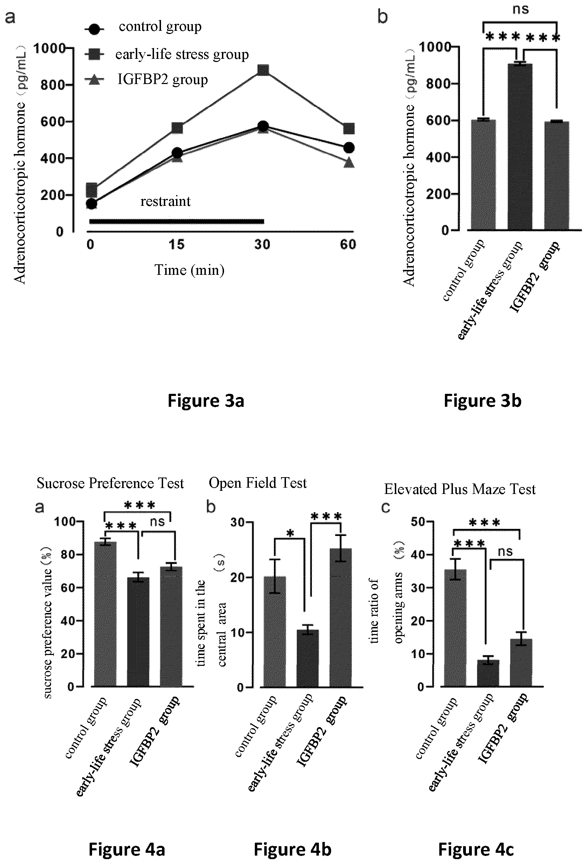

Therefore, the above ethology assessment have demonstrated the effect of IGFBP2 on anxiety and depression-like behavior caused by early-life stress. To further demonstrate the accuracy of this effect, adrenocorticotropic hormone was detected in animal serum, with 4 animals in each group. The changes in the content of adrenocorticotropic hormone after stress in adulthood are detected, as shown in Figures 3a-3b. Figure 3a shows the changes in serum adrenocorticotropic hormone levels at different time after restraint. Two factor analysis of variance, where *** represents P<0.001, ** represents P<0.001, and * represents P<0.05; Figure 3b shows the comparison of adrenocorticotropic hormone levels in animal serum among three groups after 30 minutes of restraint, One-way ANOVA, where *** represents P<0.001, ** represents P<0.001, and * represents P<0.05.

As shown in Figure 3a, the content of adrenocorticotropic hormone before restraint in the mother-infant separation group was higher than that in the other two groups (P<0.01), while there was no significant difference between the normal feeding group and the IGFBP2 intervention group. In the normal feeding group, the content of adrenocorticotropic hormone after experiencing stress was significantly higher than before, but its growth rate was significantly lower than that of the mother-infant separation group. After intervention with IGFBP2 in adolescence, animals experiencing mother-infant separation also showed a decreasing trend in growth. This result further confirms that IGFBP2 can significantly improve psychic trauma caused by early-life stress.

Meanwhile, comparing the levels of adrenocorticotropic hormone in the three groups of animals restrained for 30 minutes, as shown in Figure 3b, it was found that the mother-infant separation group had significantly higher levels than the other two groups, while there was no significant difference between the normal feeding group and the IGFBP2 intervention group. Proved the intervention effect of IGFBP2 on mental disorders caused by early-life stress.

Therefore, Example 2 indicates that IGFBP2 intervention in adolescence can significantly improve depression and anxiety-like behavior caused by early-life stress.

### Example 3

### Adult intervention

The animals were divided into three groups: the control group (normal feeding group), the mother-infant separation group, and the IGFBP2 intervention in adulthood group. The number of animals in three groups is greater than or equal to 10. As shown in Figure 1, the intervention time of IGFBP2 in adulthood is PND 63, and the intervention method is tail vein injection. The intervention dose is 1 µ g/kg, and the intervention frequency is 1 time. After the intervention, the animals were placed back in the breeding cage and subjected to ethology assessment the next day, PND64.

Ethology assessment is used to verify the intervention effect of IGFBP2 on psychic trauma caused by early-life stress. All ethology assessment were conducted during rat light exposure. Before conducting ethology assessment, the rats were placed in a behavioral room and acclimated for 2 hours. During each experiment, wipe each mouse with 70% alcohol to avoid the impact of odor on the next mouse. Handle rats for a few minutes every day before ethology assessment, and adapt for at least 4 days. Ethology assessment include Sucrose Preference Test for evaluating depression-like behavior, as well as Open Field Test and Elevated Plus Maze Test for evaluating anxiety-like behavior.

Similar to the ethology assessment of puberty intervention, the first step is to conduct Sucrose Preference Test to detect depression-like behavior in animals, followed by Open Field Test and Elevated Plus Maze Test to detect anxiety-like behavior in animals. As shown in Figures 4a to 4c, the effect of IGFBP2 intervention in adulthood on anxiety and depression-like behavior in animals of adulthood is shown. Figure 4a shows Sucrose Preference Test, with sucrose preference value%=sucrose consumption/( sucrose consumption + water consumption) x100; Figure 4b shows Open Field Test, with a total time of 10 minutes and the analysis data shows the time the animals spent in the central area; Figure 4c shows Elevated Plus Maze Test, with an open arm time ratio %=open arm time/(open arm time + closed arm time) x100. One-way ANOVA, *** represents P<0.001, ** represents P<0.001, * represents P<0.05, and ns represents no significant difference between the two groups.

As shown in Figure 4a, the results of Sucrose Preference Test showed that the sucrose preference values (66.25%) of animals in the mother-infant separation group (early-life stress group) were significantly lower than those of the normal feeding group (87.65%, One-way ANOVA, P<0.001), while the sucrose preference value (72.88%) of animals in the IGFBP2 intervention group increased compared to the mother-infant separation group, but there was no significant difference (P=0.14). This result indicates that IGFBP2 intervention in adulthood does not significantly improve depression-like behavior caused by early-life stress.

As shown in Figure 4b, the results of Open Field Test showed that the time (10.49 s)spent in the central area of the mother-infant separation group (early-life stress group) was significantly lower than that of the normal feeding group (20.18 s, One-way ANOVA, P=0.0151). After the IGFBP2 intervention, the time spent in the central area of the mother-infant separation group was significantly increased (25.23 s, One-way ANOVA, P=0.0003), and there was no difference between the mother-infant separation group and the normal feeding group (P=0.2764). This result indicates that IGFBP2 intervention in adulthood can significantly improve anxiety-like behavior caused by mother-infant separation.

In addition, comparing the motor distance of the three groups of animals, it was found that there was no significant difference in motor distance between the three groups, indicating that early-life stress and IGFBP2 intervention did not affect the motor function of the animals.

As shown in Figure 4c, the results of Elevated Plus Maze Test showed that the time ratio of opening arms in the normal feeding group (35.36%) was significantly longer than that in the mother-infant separation group (early-life stress group, 8.070%). However, the IGFBP2 intervention in adulthood improved the time of opening arms in mother-infant separation animals, but there was no significant difference between the two (14.56%, One-way ANOVA, P=0.1198). This result further confirms that IGFBP2 can effectively improve anxiety-like behavior caused by early-life stress.

Therefore, Example 3 demonstrates that intervention in adulthood can improve anxiety-like behavior caused by early-life stress.

In this specification, the present invention has been described with reference to its specific embodiments. However, it is evident that various modifications and transformations can still be made without departing from the spirit and scope of the present invention. Therefore, the specification is considered explanatory rather than restrictive.

## Claims

1. An application of insulin-like growth factor-binding protein 2 in preparation of drugs for resisting mental disorders caused by early-life stress.

2. The application according to claim 1, **characterized in that** the mental disorders comprises anxiety and depression-like behavior.
